# FASCICULE DE BREVET EUROPEEN

(11) **EP 2 961 728 B1**
(45) Date de publication et mention de la délivrance du brevet: **13.12.2017**
(21) Numéro de dépôt: 14713169.2
(22) Date de dépôt: 24.02.2014
(51) Int. Cl.: C07C 67/03, C07C 67/54

(54) **PROCÉDÉ DE PRODUCTION D'ACRYLATE DE 2-PROPYL HEPTYLE PAR TRANSESTÉRIFICATION**
VERFAHREN ZUR HERSTELLUNG VON 2-PROPYLHEPTYLACRYLAT DURCH UMESTERUNG
PROCESS FOR PRODUCING 2-PROPYLHEPTYL ACRYLATE BY TRANSESTERIFICATION

(30) Priorité: 01.03.2013 FR 1351838
(43) Date de publication de la demande: 06.01.2016
(73) Titulaire: Arkema France, 92700 Colombes (FR)
(72) Inventeur: RIONDEL, Alain, 77178 Saint Pathus (FR); GRAIRE, Coralie, 69290 Grezieu-la-Varenne (FR)
(74) Mandataire: Bonnel, Claudine
(86) Numéro de dépôt international: PCT/FR2014/050367
(87) Numéro de publication internationale: WO 2014/131970

(56) Documents cités:
- JP-A- H0 570 403
- US-B2- 7 268 251

## Description

### DOMAINE DE L'INVENTION

La présente invention concerne la production d'acrylate de 2-propyl heptyle selon un procédé semi continu par transestérification.

### ARRIERE-PLAN TECHNIQUE

Il est connu de produire des esters acryliques en mettant en oeuvre une réaction de transestérification entre un acrylate d'alcool léger (dénommé acrylate léger) et un alcool lourd.

Cette réaction est une réaction catalysée équilibrée avec génération d'alcool léger, selon la formule (I) :

CH₂=CH-COOR₁ + R2-OH ⇔ CH₂=CH-COOR₂ + R₁-OH (I)

Il est nécessaire d'éliminer l'alcool léger produit au cours de la réaction pour déplacer l'équilibre dans le sens de la production de l'ester acrylique.

Cette réaction s'accompagne généralement de réactions secondaires produisant des impuretés qu'il est nécessaire d'éliminer en vue d'obtenir l'ester acrylique avec une pureté élevée satisfaisant aux exigences techniques liées à son utilisation finale en tant que monomère pour fabriquer des polymères utilisables dans de nombreux domaines d'application.

Par ailleurs, pour des raisons économiques évidentes, les produits valorisables présents dans le mélange brut réactionnel, notamment les réactifs non réagis et le catalyseur, sont dans la mesure du possible recyclés au sein du procédé.

A ces fins, on procède généralement à un processus de séparation/purification comportant un ensemble de distillations, d'extractions, et/ou décantations, qui est à la fois relativement complexe à mettre en oeuvre, notamment du fait de la présence de mélanges azéotropiques, et coûteux sur le plan énergétique.

La Société Déposante s'est intéressée plus particulièrement à la synthèse de l'acrylate de 2-propyl heptyle (acrylate d'alkyle avec une chaine en C₁₀ ramifiée) à partir d'un acrylate léger et de 2-propyl heptanol (dénommé 2-PH), ce monomère pouvant présenter des propriétés intéressantes dans le domaine des matériaux de revêtement, les peintures, les encres, et les adhésifs.

Selon le document JP05-070403, l'acrylate de 2-propyl heptyle peut être obtenu par estérification directe d'acide acrylique, ou par transestérification d'un acrylate léger tel que l'acrylate de méthyle, avec le 2-propyl heptanol en présence d'acide p-toluène sulfonique comme catalyseur et d'un inhibiteur de polymérisation, selon les méthodes bien connues de l'homme de l'art. Le méthanol formé est éliminé en continu et la réaction est suivie d'une distillation sous pression réduite en guise de purification du produit de réaction obtenu. Le document JP05-070403, ne mentionne pas précisément les étapes de purification à mettre en oeuvre, ni le niveau de pureté à atteindre pour le monomère en vue de son utilisation.

Différents procédés de transestérification pour produire des acrylates d'alkyle en C₂-C₁₂ ont déjà été décrits dans l'art antérieur.

On peut citer par exemple le document US 7,268,251 dans lequel l'effluent réactionnel de la transestérification est traité de la façon suivante :
- soit on sépare tout d'abord la majeure partie de l'ester acrylique recherché et on l'isole ensuite du catalyseur utilisé par distillation (séparation de catalyseur),
- soit on l'isole tout d'abord du catalyseur utilisé par distillation (séparation de catalyseur) et ensuite on sépare la majeure partie de l'ester acrylique,
- et ensuite on sépare par distillation du mélange obtenu, les composés à point d'ébullition inférieur à celui de l'ester acrylique recherché (séparation des substances à bas point d'ébullition) et ensuite on distille l'ester acrylique (distillation à l'état pur).

Ce procédé nécessite la mise en oeuvre d'au moins quatre colonnes de distillation ou de rectification, dont un évaporateur pour séparer le catalyseur, généralement un alcoolate de titane.

Même si le procédé décrit dans le document US 7,268,251 concerne la fabrication d'acrylates d'alkyle par transestérification à partir d'un acrylate d'alkyle et d'un alcool présentant une longueur de chaine supérieure d'au moins un carbone par rapport à la chaine alkyle de l'acrylate de départ, ce procédé n'est illustré qu'avec la fabrication d'acrylate de diméthylaminoéthyle à partir de diméthylaminoéthanol et d'acrylate de méthyle ou d'acrylate d'éthyle dans une cascade de deux réacteurs.

Il s'avère que le procédé décrit dans le document US 7,268,251 est compliqué à mettre en oeuvre à l'échelle industrielle, du fait de l'optimisation des conditions opératoires de la succession des quatre éléments de distillation/rectification, pour obtenir un produit d'une grande pureté et une productivité satisfaisante.

Le document US 6,977,310 décrit un procédé de fabrication en continu d'esters alkyliques d'acide (méth)acrylique à partir de (méth)acrylate de méthyle et d'un alcool en C₂-C₁₂, en présence d'un titanate de tétraalkyle comme catalyseur de transestérification. Ce procédé consiste à soumettre le mélange réactionnel à une distillation sous pression réduite séparant les composés aisément volatils (réactifs non réagis) ; puis, la fraction résultante sortant en pied de colonne, comprenant l'ester produit, le catalyseur, les inhibiteurs de polymérisation et les produits secondaires à point d'ébullition élevé, est envoyée vers un étage de distillation sous vide. Cet étage de distillation sous vide comprend notamment un évaporateur à film, combiné avec une colonne de distillation pour une élimination complète des produits à point d'ébullition élevé dans l'ester produit. L'ester produit est récupéré en tête avec une grande pureté.

Selon ce procédé, illustré uniquement avec la fabrication de méthacrylate de butyle ou de méthacrylate d'isobutyle, le méthacrylate recherché se trouve dans le flux de pied de la première colonne de distillation sous pression réduite avant d'être séparé du catalyseur puis purifié.

Les procédés de l'art antérieur utilisant un titanate d'alkyle tel que titanate de tétraéthyle, titanate de tétrabutyle, titanate de tétra(2-éthylhexyle) comme catalyseur de transestérification ne sont pas directement applicables à la fabrication d'acrylate d'alkyle à chaine longue, par exemple l'acrylate de 2-propyl heptyle, par réaction de transestérification d'un acrylate léger avec du 2-propyl heptanol. En effet, la transestérification des titanates, soit avec l'alcool léger libéré au cours de la réaction (méthanol ou éthanol), soit avec le 2-propyl heptanol de départ, provoque l'apparition d'impuretés telles que acrylate de butyle ou acrylate de 2-éthylhexyle, dans le mélange réactionnel ou dans le mélange azéotropique ester léger /alcool léger et complique la purification de l'acrylate de 2-propyl heptyle.

La Société Déposante a cherché à résoudre les différents problèmes des procédés précités, en particulier ceux liés à la mise en oeuvre de 2-propyl heptanol dans la réaction de transestérification catalysée par un titanate d'alkyle, et a découvert un procédé de fabrication simplifié pour produire de l'acrylate de 2-propyl heptyle de très haute pureté avec un rendement élevé, tout en incluant le recyclage des produits valorisables tels que les réactifs non réagis et le catalyseur, et présentant ainsi une productivité compatible avec une fabrication à l'échelle industrielle.

La solution proposée consiste à utiliser du titanate d'éthyle en solution dans le 2-propyl heptanol ou du titanate de 2-propyl heptyle comme catalyseur de transestérification, et à mettre en oeuvre un train de purification ne comportant qu'une seule colonne de distillation et un évaporateur à film.

Par ailleurs, les inventeurs ont trouvé que les dérivés de l'étain, en particulier l'oxyde de dibutyl étain, comme catalyseurs de transestérification, permettent également de pallier les inconvénients des procédés précités et sont utilisables pour produire de l'acrylate de 2-propyl heptyle dans un procédé de transestérification comportant un train de purification simplifié avec une seule colonne de distillation et un évaporateur à film.

### RESUME DE L'INVENTION

La présente invention a pour objet un procédé de production d'acrylate de 2-propyl heptyle par réaction de transestérification entre un acrylate d'alcool à chaine alkyle linéaire en C₁-C₄ et du 2-propyl heptanol en présence d'un titanate d'alkyle comme catalyseur de transestérification et d'au moins un inhibiteur de polymérisation, le mélange azéotropique composé d'acrylate d'alcool à chaine alkyle linéaire en C₁-C₄ et d'alcool linéaire en C₁-C₄ généré par la réaction de transestérification étant soutiré en continu pendant la réaction et le mélange réactionnel étant soumis à un traitement de purification afin d'obtenir un acrylate de 2-propyl heptyle de pureté élevée, procédé caractérisé en ce que :
- on choisit le catalyseur parmi le titanate d'éthyle en solution dans le 2-propyl heptanol et le titanate de 2-propyl heptyle ;
- on adresse à une colonne de distillation (C1) sous pression réduite, le mélange brut réactionnel comprenant l'acrylate de 2-propyl heptyle recherché avec, comme produits légers, l'acrylate d'alcool à chaine alkyle linéaire en C₁-C₄ n'ayant pas réagi et des traces de 2-propyl heptanol, et comme produits lourds, le catalyseur, le(s) inhibiteur(s) de polymérisation ainsi que des sous-produits lourds de réaction, et on effectue, dans ladite colonne (C1), une distillation permettant d'obtenir :
   o en tête, un flux composé essentiellement d'acrylate d'alcool à chaine alkyle linéaire en C₁-C₄ n'ayant pas réagi avec des traces de 2-propyl heptanol, et
   o en pied, un flux composé essentiellement d'acrylate de 2-propyl heptyle recherché, avec le catalyseur, le(s) inhibiteur(s) de polymérisation ainsi que des sous-produits lourds de réaction, et des traces de composés légers ; puis
- on adresse le flux de pied de la colonne de distillation (C1) à un évaporateur à film (E) sous pression réduite, permettant de séparer :
   o en tête, l'acrylate de 2-propyl heptyle pur recherché ; et
   o en pied, le catalyseur, le(s) inhibiteur(s) de polymérisation ainsi que les sous-produits lourds de réaction.

Dans la présente description, « acrylate d'alcool à chaîne alkyle linéaire en C₁-C₄ » pourra être désigné aussi par « acrylate d'alcool léger » ou « acrylate léger » de même signification, et « alcool linéaire en C₁-C₄ » pourra être désigné aussi par « alcool léger ».

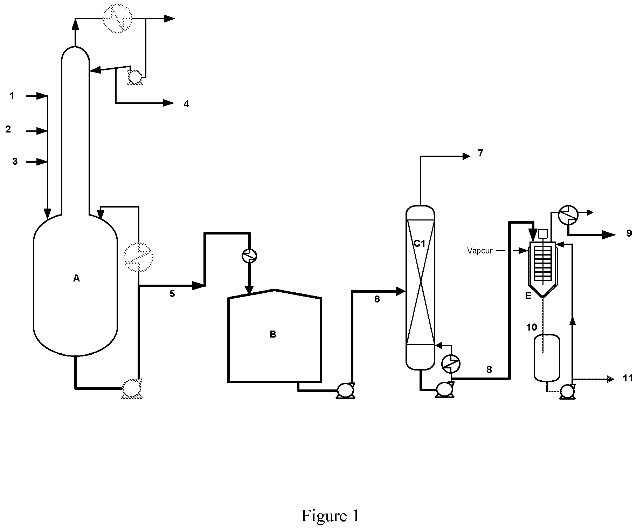

L'invention est maintenant décrite plus en détail et de façon non limitative dans la description qui suit, en référence à la figure unique annexée qui représente de manière schématique une installation permettant de mettre en oeuvre le procédé selon l'invention.

### DESCRIPTION DETAILLEE

Le procédé de production d'acrylate de 2-propyl heptyle selon l'invention peut être un procédé en batch, en continu, ou en semi-continu, c'est-à-dire avec la partie réactionnelle en batch et la partie purification en continu. De préférence, le procédé selon l'invention est semi-continu.

L'alcool 2-propyl heptanol utilisé dans le procédé selon l'invention peut être représenté sous la forme : C₅H₁₁CH(C₃H₇)CH₂OH avec le groupe C₅H₁₁ pouvant représenter n-C₅H₁₁, C₂H₅CH(CH₃)CH₂ ou CH₃CH(CH₃)CH₂CH₂.

Par la dénomination simplifiée 2-PH dans la suite de la description, on entend le 2-propyl heptanol sous la forme de chacun de ces isomères seuls ou en mélange. Le 2-PH peut contenir en outre d'autres isomères en faible teneur, tels que 2-isopropyl heptanol, 2-isopropyl-4-méthyl hexanol, 2-isopropyl-5-méthyl hexanol ou 2-propyl-4,4-diméthyl pentanol.

Le 2-PH peut être produit de différentes manières, par exemple par aldolisation de n-valéraldéhyde produit par hydroformylation de butènes, déshydratation de l'alcool obtenu en 2-propyl-2-heptanal, suivie d'une hydrogénation.

Le 2-PH peut être également obtenu par condensation de 1-pentanol (sous la forme d'un mélange de méthyl butanols) en présence de KOH à une température élevée selon une réaction de Guerbet.

Selon l'invention, le 2-PH contient majoritairement du 2-propyl heptanol. En général, le 2-PH est un mélange comprenant :
- de 70 à 99%, de préférence de 80 à 95% de 2-propyl heptanol n-C₅H₁₁CH(C₃H₇)CH₂H et
- de 1 à 30%, de préférence de 5 à 20% d'un mélange de 4-méthyl 2-propyl hexanol C₂H₅CH(CH₃)CH₂CH(C₃H₇)CH₂OH et de 5-méthyl 2-propyl hexanol CH₃CH(CH₃)CH₂CH₂CH(C₃H₇)CH₂OH.
Le 2-propyl heptanol est commercialisé notamment par la société BASF.

Comme acrylate d'alcool à chaine alkyle en C₁-C₄ mis en oeuvre comme matière première dans le procédé selon l'invention, on utilise l'acrylate de méthyle, l'acrylate d'éthyle ou l'acrylate de butyle, de préférence l'acrylate d'éthyle.

L'acrylate d'alcool léger est obtenu par estérification directe de l'acide acrylique essentiellement produit sur le plan industriel à partir de propylène, avec un alcool léger, généralement le méthanol, l'éthanol ou le butanol.

L'invention s'étend à l'utilisation d'un acrylate d'alcool léger dérivé d'acide acrylique d'origine renouvelable, pouvant être en particulier obtenu à partir de glycérol, selon un procédé comportant une première étape de déshydratation du glycérol en acroléine suivie d'une étape d'oxydation en phase gaz de l'acroléine ainsi obtenue ; ou obtenu par déshydratation des acides 2-hydroxypropionique (acide lactique) ou 3-hydroxypropionique et de leurs esters.

L'invention s'étend également à l'utilisation d'un acrylate d'alcool léger dérivé d'un alcool bio-sourcé, tel que le bioéthanol.

D'une manière générale, la réaction de transestérification est réalisée dans un réacteur agité (A), chauffé par un échangeur externe et surmonté d'une colonne à distiller, en présence d'un excès d'acrylate d'alcool léger, en particulier avec un rapport molaire acrylate d'alcool léger / 2-PH pouvant aller de 1 à 3, de préférence compris entre 1,3 et 1,8.

Le catalyseur de transestérification est le titanate d'éthyle en solution dans du 2-PH, par exemple une solution à 90% de titanate d'éthyle dans du 2-PH, ou le titanate de 2-propyl heptyle, obtenu préalablement par réaction à 100°C du titanate d'éthyle avec le 2-PH, de préférence on utilise le titanate d'éthyle en solution dans du 2-PH.

L'invention s'étend à l'utilisation de dérivés de l'étain, comme catalyseurs de transestérification, tels que les oxydes de dialkyl étain, la chaîne alkyle linéaire ou ramifiée, ayant de 1 à 8 atomes de carbone. On peut citer comme exemples les oxydes de dialkyl étain de chaîne alkyle linéaire ayant de 1 à 4 atomes de carbone, tels que l'oxyde de diméthyl étain, l'oxyde de diéthyl étain, ou plus particulièrement l'oxyde de n-dibutyl étain (DBTO).

On utilise le catalyseur à raison de 5.10⁻⁴ à 5.10⁻² mole par mole de 2-PH, de préférence à raison de 10⁻³ à 10⁻² mole par mole de 2-PH.

On conduit généralement la réaction de transestérification dans le réacteur (A) à une pression comprise entre 200 mm Hg (0,27 10⁵ Pa) et la pression atmosphérique, et à une température allant de 90°C à 130°C, de préférence de 95°C à 110°C.

La réaction est effectuée en présence d'un ou plusieurs inhibiteurs de polymérisation qui sont introduits dans le réacteur, à raison de 10 à 5000 ppm par rapport au mélange brut réactionnel, et de préférence de 200 à 1000 ppm.

Comme inhibiteurs de polymérisation utilisables, on peut citer par exemple la phénothiazine, l'hydroquinone, l'éther monométhylique d'hydroquinone (EMHQ), le diterbutyl para-crésol (BHT), le TEMPO (2,2,6,6-tétraméthyl-1-piperidinyloxy), le di-tertiobutylcatéchol, ou les dérivés du TEMPO, tel que le 4-hydroxy TEMPO (4-OHTEMPO), seuls ou leurs mélanges en toutes proportions. De préférence, on utilise l'éther monométhylique d'hydroquinone (EMHQ) comme inhibiteur de polymérisation.

Un ajout supplémentaire d'inhibiteur de polymérisation est généralement effectué au niveau de la colonne de distillation surmontant le réacteur et lors du traitement ultérieur de purification, en particulier au niveau de la colonne de distillation (C1), et dans le produit final en aval du condenseur de tête de l'évaporateur à film.

L'alcool linéaire en C₁-C₄ (généralement méthanol ou éthanol) formé par la réaction de transestérification est entraîné en continu par distillation dans la colonne surmontant le réacteur sous forme d'un mélange azéotropique avec l'acrylate d'alcool léger. Ce mélange est avantageusement recyclé à l'unité de synthèse de l'acrylate à chaine alkyle en C₁-C₄. Cette unité de synthèse procède selon une estérification directe d'acide acrylique avec l'alcool linéaire en C₁-C₄.

Après réaction avec un temps de séjour dans le réacteur généralement compris entre 3 et 6 heures, le mélange brut réactionnel 5 contient l'acrylate de 2-propyl heptyle recherché avec, comme produits légers, l'acrylate d'alcool léger n'ayant pas réagi et des traces de 2-HP, et comme produits lourds, le catalyseur, le ou les inhibiteurs de polymérisation ainsi que des sous-produits lourds de réaction.

Dans le cas où le procédé est mis en oeuvre selon un mode semi-continu, le mélange réactionnel est stocké dans un réservoir tampon (B) avant d'être soumis au traitement de purification.

Le traitement de purification comporte une colonne de distillation (C1), généralement dénommée aussi colonne de stripping par l'homme de l'art, dans laquelle de l'air est envoyée en pied, et un évaporateur à film, afin d'obtenir dans des fractions séparées, l'acrylate de 2-propyl heptyle pur, l'acrylate d'alcool léger en excès présent dans le mélange réactionnel, et le catalyseur avec le(s) inhibiteur(s) de polymérisation et les sous-produits lourds de réaction.

La colonne de distillation (C1) fonctionne généralement sous une pression allant de 10 à 50 mm Hg (0,013 10⁵ Pa à 0,067 10⁵ Pa) à une température de pied allant de 90°C à 150°C.

Le flux 7 de tête de colonne (C1) est constitué principalement d'acrylate d'alcool léger non réagi avec des traces de 2-PH.

Ce flux 7 est de préférence condensé et récupéré pour être recyclé à la réaction de transestérification dans le réacteur (A). Cependant, ce flux, présent en faible quantité, peut être simplement éliminé dans les effluents aqueux du procédé via une colonne de lavage des évents.

Le flux 8 de pied de colonne (C1) est constitué principalement d'acrylate de 2-propyl heptyle recherché avec le catalyseur, les inhibiteurs de polymérisation et les sous-produits lourds et peut contenir des traces résiduelles de composés légers (réactifs non réagis).

Ce flux 8 est envoyé sur un évaporateur à film (E) pour séparer le catalyseur du produit fini, la séparation du catalyseur dans le mélange « strippé » ne nécessitant pas la mise en oeuvre d'une colonne de distillation. On sépare en tête l'acrylate de 2-propyl heptyle purifié 9, et en pied une fraction 10 comportant le catalyseur, les inhibiteurs de polymérisation ainsi que les sous-produits lourds de réaction.

Une partie 11 de ce flux 10 peut être de façon optionnelle recyclée au niveau de la réaction dans le réacteur (A), le restant étant éliminé et incinéré afin d'éviter une trop grande accumulation de sous-produits lourds au niveau du réacteur.

Comme évaporateur à film, on peut notamment utiliser un évaporateur à film tombant ou un évaporateur à film raclé. Ce type d'évaporateur présente l'avantage d'avoir un temps de séjour réduit ce qui limite la formation de composés lourds supplémentaires en aval de la section réaction. Cet évaporateur se compose essentiellement d'une partie cylindrique chauffée par double enveloppe, d'une partie supérieure servant à la séparation des vapeurs et d'un rotor tournant à grande vitesse. Le flux à traiter est étalé sur toute la surface de chauffe sous forme d'un film à grande turbulence. Les vapeurs qui se forment montent à contre-courant vers le haut de l'appareil. Les produits non évaporés, essentiellement le catalyseur, les inhibiteurs de polymérisation et les sous-produits lourds, atteignent la partie inférieure de l'évaporateur et sont évacués sous forme du flux 10. Le flux gazeux 9, en tête de l'évaporateur, est constitué du produit recherché purifié.

L'évaporateur à film fonctionne dans des conditions de pression de l'ordre de 10 mbar et à une température de l'ordre de 160°C.

Le procédé de fabrication d'acrylate de 2-propyl heptyle selon l'invention présente une productivité compatible avec une fabrication à l'échelle industrielle et conduit à un acrylate de 2-propyl heptyle de pureté supérieure à 99,5% répondant aux exigences de pureté liées à son utilisation finale, notamment quant à la possibilité d'utiliser ce monomère pour la fabrication de latex à faible teneur en composés organiques volatiles dans les domaines par exemple des adhésifs, revêtements, peintures ou encres.

### PARTIE EXPERIMENTALE

Dans les exemples, les pourcentages sont indiqués en poids sauf indication contraire et les abréviations suivantes ont été utilisées :
AE : acrylate d'éthyle
2-PH : 2-propyl heptanol
A2PH : acrylate de 2-propyl heptyle
EMHQ : éther méthylique d'hydroquinone

### Exemple 1 (selon l'invention)

Dans un réacteur A parfaitement agité de 1 L, chauffé par un échangeur externe de type thermo siphon, surmonté d'une colonne à distiller à garnissage ordonné de type Multiknit de 12 plateaux théoriques, on charge 387 g de 2-propyl heptanol (2-PH, flux 1), 490 g d'acrylate d'éthyle (AE, flux 2), 1,31 g de titanate d'éthyle en solution dans du 2-PH, (mélange à 85% dans le 2-PH) avec 0,13 g d'EMHQ (flux 3), sous bullage d'air pour stabiliser le milieu réactionnel. En tête de la colonne à distiller, on introduit un mélange à 500 ppm d'EMHQ dans l'AE (non représenté).

On porte le bain d'huile à une température de 125°C et on distille en continu l'azéotrope AE/ éthanol (4).

La pression en tête de colonne est régulée pour que la température dans le réacteur ne dépasse pas 95°C.

Au cours de cette étape, la pression varie entre 631-324 mbars et on recueille 166 g d'azéotrope AE/éthanol contenant 69% massique d'éthanol. La durée de la réaction est 3 heures.

Le brut réactionnel 5 (masse 652 g) a la composition massique suivante :
- AE : 20,5%
- 2-PH (somme des isomères) : 0,27%
- A2PH somme des isomères) : 78,47%
- Catalyseur + stabilisant + impuretés : 0,76%

Le brut réactionnel est stocké dans un réservoir tampon B qui alimente en continu une colonne de distillation (C1) en partie haute via un flux 6.

La colonne C1 est une colonne à garnissage ordonné type multiknit de 7 plateaux théoriques opérant sous pression réduite, et chauffée par un échangeur externe type thermo-siphon. En tête de colonne C1, on introduit un mélange à 500 ppm d'EMHQ dans l'AE (non représenté).

La colonne C1 fonctionne sous vide (20-30 mbars, 100°C maxi) et sépare :
- en tête un flux 7 à 99,5% d'AE (138g/h) ; et
- en pied un flux 8 d'A2PH brut (514g/h) de composition massique :
   AE : 0,1%
   2-PH (somme des isomères) : 0,25%
   A2PH (somme des isomères) : 98,69%
   Catalyseur + stabilisant + impuretés : 0,96%

Le flux 7 d'AE est envoyé vers un réservoir intermédiaire (non représenté sur le schéma) pour être recyclé à la réaction.

Le flux 8 d'A2PH brut est envoyé en continu vers un évaporateur à film E qui opère sous pression réduite (10 mbars, 160°C).

L'évaporateur à film sépare :
- en tête un flux 9 d'A2PH purifié (463g/h) de composition massique :
   A2PH (somme des isomères) : 99,57%
   AE : 0,15%
   2-PH (somme des isomères) : 0,27%
   Stabilisant EMHQ : 250 ppm
- en pied un flux 10 comprenant un mélange de sous-produits lourds et le catalyseur (52g/h) qui est optionnellement recyclé pour partie 11 à la réaction, le reste étant éliminé.

### Exemple 2 (comparatif)

La même synthèse que celle décrite à l'exemple 1 est conduite en utilisant le titanate de butyle comme catalyseur.

Dans ce cas, le flux de tête 7 d'AE issu de la colonne C1 contient 10% d'acrylate de butyle (ABU), ce qui le rend impropre au recyclage en l'état à l'étape réactionnelle, et nécessite une colonne à distiller supplémentaire pour séparer l'AE de l'acrylate de butyle.

### Exemple 3 (comparatif)

La même synthèse que celle décrite à l'exemple 1 est conduite en utilisant le titanate de 2-éthylhexyle comme catalyseur.

Dans ce cas, la pureté de l'A2PH dans le flux 9 en tête de l'évaporateur à film n'est plus que de 97,5% en raison de la présence de 2% d'acrylate de 2-éthylhexyle. Cette qualité d'A2PH n'offre pas les mêmes performances dans les adhésifs sensibles à la pression qu'un A2PH de pureté 99,5%.

## Revendications

1. Procédé de production d'acrylate de 2-propyl heptyle par réaction de transestérification entre un acrylate d'alcool à chaine alkyle linéaire en C₁-C₄ et du 2-propyl heptanol en présence d'un titanate d'alkyle comme catalyseur de transestérification et d'au moins un inhibiteur de polymérisation, le mélange azéotropique composé d'acrylate d'alcool à chaine alkyle linéaire en C₁-C₄ et d'alcool linéaire en C₁-C₄ généré par la réaction de transestérification étant soutiré en continu pendant la réaction et le mélange réactionnel étant soumis à un traitement de purification afin d'obtenir un acrylate de 2-propyl heptyle de pureté élevée, procédé **caractérisé en ce que** :
- on choisit le catalyseur parmi le titanate d'éthyle en solution dans le 2-propyl heptanol et le titanate de 2-propyl heptyle ;
- on adresse à une colonne de distillation (C1) sous pression réduite, le mélange brut réactionnel comprenant l'acrylate de 2-propyl heptyle recherché avec, comme produits légers, l'acrylate d'alcool à chaine alkyle linéaire en C₁-C₄ n'ayant pas réagi et des traces de 2-propyl heptanol, et comme produits lourds, le catalyseur, le(s) inhibiteur(s) de polymérisation ainsi que des sous-produits lourds de réaction, et on effectue, dans ladite colonne (C1), une distillation permettant d'obtenir :
∘ en tête, un flux composé essentiellement d'acrylate d'alcool à chaine alkyle linéaire en C₁-C₄ n'ayant pas réagi avec des traces de 2-propyl heptanol, et
∘ en pied, un flux composé essentiellement d'acrylate de 2-propyl heptyle recherché, avec le catalyseur, le(s) inhibiteur(s) de polymérisation ainsi que des sous-produits lourds de réaction, et des traces de composés légers ; puis
- on adresse le flux de pied de la colonne de distillation (C1) à un évaporateur à film (E) sous pression réduite, permettant de séparer :
∘ en tête, l'acrylate de 2-propyl heptyle pur recherché ; et
∘ en pied, le catalyseur, le(s) inhibiteur(s) de polymérisation ainsi que les sous-produits lourds de réaction.

2. Procédé selon la revendication 1 **caractérisé en ce que** le catalyseur est le titanate d'éthyle en solution dans le 2-propyl heptanol.

3. Procédé selon la revendication 1 ou 2 **caractérisé en ce qu'**il est opéré en mode batch, continu ou semi-continu, de préférence en mode semi-continu.

4. Procédé selon l'une quelconque des revendications précédentes **caractérisé en ce que** le 2-propyl heptanol est un mélange comprenant :
- de 70 à 99%, de préférence de 80 à 95% de 2-propyl heptanol n-C₅H₁₁CH(C₃H₇)CH₂OH et
- de 1 à 30%, de préférence de 5 à 20% d'un mélange de 4-méthyl 2-propyl hexanol C₂H₅CH(CH₃)CH₂CH(C₃H₇)CH₂OH et de 5-méthyl 2-propyl hexanol CH₃CH(CH₃)CH₂CH₂CH(C₃H₇)CH₂OH.

5. Procédé selon l'une quelconque des revendications précédentes **caractérisé en ce que** l'acrylate d'alcool à chaine alkyle linéaire en C₁-C₄ est l'acrylate d'éthyle.

6. Procédé selon l'une quelconque des revendications précédentes **caractérisé en ce que** l'évaporateur à film est un évaporateur à film tombant ou à film raclé.

7. Procédé selon l'une quelconque des revendications précédentes **caractérisé en ce que** le flux de tête de la colonne C1 est recyclé à la réaction.

8. Procédé selon l'une quelconque des revendications précédentes **caractérisé en ce que** le flux séparé en pied de l'évaporateur à film est recyclé au moins en partie à la réaction.

9. Procédé selon l'une quelconque des revendications précédentes **caractérisé en ce que** le mélange azéotropique composé d'acrylate d'alcool à chaine alkyle linéaire en C₁-C₄ et d'alcool linéaire en C₁-C₄ généré par la réaction de transestérification est recyclé à l'unité de synthèse de l'acrylate d'alcool à chaine alkyle linéaire en C₁-C₄.

10. Procédé selon l'une quelconque des revendications précédentes **caractérisé en ce que** l'inhibiteur de polymérisation est l'éther monométhylique d'hydroquinone (EMHQ).

## Patentansprüche

1. Verfahren zur Herstellung von 2-Propylheptylacrylat durch Umesterungsreaktion zwischen einem Acrylat eines Alkohols mit linearer C₁-C₄-Alkylkette und 2-Propylheptanol in Gegenwart eines Alkyltitanats als Umesterungskatalysator und mindestens eines Polymerisationshemmers, wobei das azeotrope Gemisch, welches sich aus Acrylat eines Alkohols mit linearer C₁-C₄-Alkylkette und aus linearem C₁-C₄-Alkohol zusammensetzt und durch die Umesterungsreaktion entsteht, während der Reaktion kontinuierlich abzogen wird und die Reaktionsmischung einer Aufreinigungsbehandlung unterzogen wird, um ein 2-Propylheptylacrylat von hoher Reinheit zu erhalten, wobei das Verfahren **dadurch gekennzeichnet ist, dass**
- der Katalysator aus Ethyltitanat, das in 2-Propylheptanol gelöst ist, und 2-Propylheptyltitanat ausgewählt wird,
- einer Destillationskolonne (C1), die unter vermindertem Druck steht, die rohe Reaktionsmischung zugeführt wird, welche das angestrebte 2-Propylheptylacrylat gemeinsam mit nicht umgesetztem Acrylat eines Alkohols mit linearer C₁-C₄-Alkylkette und Spuren an 2-Propylheptanol als leichtflüchtige Stoffe und mit dem Katalysator, dem/den Polymerisationshemmer(n) sowie den schwerflüchtigen Reaktionsnebenprodukten als schwerflüchtige Stoffe umfasst, und in der Kolonne (C1) eine Destillation durchgeführt wird, mittels derer Folgendes erhalten werden kann:
∘ kopfseitig ein Stoffstrom, der sich im Wesentlichen aus nicht umgesetztem Acrylat eines Alkohols mit linearer C₁-C₄-Alkylkette mit Spuren von 2-Propylheptanol zusammensetzt, und
∘ sumpfseitig ein Stoffstrom, der sich im Wesentlichen aus dem angestrebten 2-Propylheptylacrylat mit dem Katalysator, dem/den Polymerisationshemmer(n) sowie den schwerflüchtigen Reaktionsnebenprodukten und aus Spuren von leichtflüchtigen Verbindungen zusammensetzt; woraufhin
- der sumpfseitige Stoffstrom der Destillationskolonne (C1) einem Dünnschichtverdampfer (E) zugeführt wird, der unter vermindertem Druck steht und mittels dessen Folgendes voneinander getrennt werden kann:
∘ kopfseitig das angestrebte reine 2-Propylheptylacrylat; und
∘ sumpfseitig der Katalysator, der/die Polymerisationshemmer sowie die schwerflüchtigen Reaktionsnebenprodukte.

2. Verfahren nach Anspruch 1, **dadurch gekennzeichnet, dass** es sich bei dem Katalysator um Ethyltitanat handelt, das in 2-Propylheptanol gelöst ist.

3. Verfahren nach Anspruch 1 oder 2, **dadurch gekennzeichnet, dass** es chargenweise, kontinuierlich oder halbkontinuierlich betrieben wird, vorzugsweise halbkontinuierlich.

4. Verfahren nach einem beliebigen der vorhergehenden Ansprüche, **dadurch gekennzeichnet, dass** der 2-Propylheptanol einer Mischung entspricht, die Folgendes umfasst:
- 70 bis 99 %, vorzugsweise 80 bis 95 %, an 2-Propylheptanol n-C₅H₁₁CH(C₃H₇)CH₂OH und
- 1 bis 30 %, vorzugsweise 5 bis 20 %, einer Mischung aus 4-Methyl-2-propylhexanol C₂H₅CH(CH₃)CH₂CH(C₃H₇)CH₂OH und aus 5-Methyl-2-propylhexanol CH₃CH (CH₃) CH₂CH₂CH (C₃H₇) CH₂OH.

5. Verfahren nach einem beliebigen der vorhergehenden Ansprüche, **dadurch gekennzeichnet, dass** es sich bei dem Acrylat eines Alkohols mit linearer C₁-C₄-Alkylkette um Ethylacrylat handelt.

6. Verfahren nach einem beliebigen der vorhergehenden Ansprüche, **dadurch gekennzeichnet, dass** es sich bei dem Dünnschichtverdampfer um einen Fallfilm- oder Streichfilmverdampfer handelt.

7. Verfahren nach einem beliebigen der vorhergehenden Ansprüche, **dadurch gekennzeichnet, dass** der kopfseitige Stoffstrom der Kolonne C1 in den Reaktionsansatz zurückgeführt wird.

8. Verfahren nach einem beliebigen der vorhergehenden Ansprüche, **dadurch gekennzeichnet, dass** der Stoffstrom, welcher sumpfseitig des Dünnschichtverdampfers abgetrennt wird, mindestens teilweise in den Reaktionsansatz zurückgeführt wird.

9. Verfahren nach einem beliebigen der vorhergehenden Ansprüche, **dadurch gekennzeichnet, dass** das azeotrope Gemisch, welches sich aus Acrylat eines Alkohols mit linearer C₁-C₄-Alkylkette und aus linearem C₁-C₄-Alkohol zusammensetzt und durch die Umesterungsreaktion entsteht, in den Einheit zurückgeführt wird, die der Synthese des Acrylats eines Alkohols mit linearer C₁-C₄-Alkylkette dient.

10. Verfahren nach einem beliebigen der vorhergehenden Ansprüche, **dadurch gekennzeichnet, dass** es sich bei dem Polymerisationshemmer um Hydrochinonmonomethylether (HCME) handelt.

## Claims

1. A process for the production of 2-propylheptyl acrylate by a transesterification reaction between an alcohol acrylate with a linear alkyl C₁-C₄ chain and 2-propylheptanol in the presence of an alkyl titanate as transesterification catalyst and of at least one polymerization inhibitor, the azeotropic mixture composed of alcohol acrylate with a linear alkyl C₁-C₄ chain and linear C₁-C₄ alcohol generated by the transesterification reaction being continuously withdrawn during the reaction and the reaction mixture being subjected to a purification treatment in order to obtain a 2-propylheptyl acrylate of high purity, which process is **characterized in that**:
- the catalyst is chosen from ethyl titanate in solution in 2-propylheptanol and 2-propylheptyl titanate;
- the crude reaction mixture comprising the desired 2-propylheptyl acrylate with, as light products, the unreacted alcohol acrylate with a linear alkyl C₁-C₄ chain and traces of 2-propylheptanol and, as heavy products, the catalyst, the polymerization inhibitor(s) and also heavy reaction byproducts is sent to a distillation column (C1) under reduced pressure, and a distillation is carried out, in said column (C1), which makes it possible to obtain:
∘ at the top, a stream essentially composed of unreacted alcohol acrylate with a linear alkyl C₁-C₄ chain with traces of 2-propylheptanol, and
∘ at the bottom, a stream essentially composed of desired 2-propylheptyl acrylate, with the catalyst, the polymerization inhibitor(s) and also heavy reaction byproducts, and traces of light compounds; then
- the bottom stream from the distillation column (C1) is sent to a film evaporator (E) under reduced pressure, making it possible to separate:
∘ at the top, the desired pure 2-propylheptyl acrylate; and
∘ at the bottom, the catalyst, the polymerization inhibitor(s) and also the heavy reaction byproducts.

2. The process as claimed in claim 1, **characterized in that** the catalyst is ethyl titanate in solution in 2-propylheptanol.

3. The process as claimed in claim 1 or 2, **characterized in that** it is carried out in batch, continuous or semicontinuous mode, preferably in semicontinuous mode.

4. The process as claimed in any one of the preceding claims, **characterized in that** the 2-propylheptanol is a mixture comprising:
- from 70 to 99%, preferably from 80 to 95%, of 2-propylheptanol n-C₅H₁₁CH(C₃H₇)CH₂OH and
- from 1 to 30%, preferably from 5 to 20%, of a mixture of 4-methyl-2-propyl-hexanol C₂H₅CH(CH₃)CH₂CH(C₃H₇)CH₂OH and 5-methyl-2-propylhexanol CH₃CH(CH₃)CH₂CH₂CH(C₃H₇)CH₂OH.

5. The process as claimed in any one of the preceding claims, **characterized in that** the alcohol acrylate with a linear alkyl C₁-C₄ chain is ethyl acrylate.

6. The process as claimed in any one of the preceding claims, **characterized in that** the film evaporator is a falling film evaporator or a wiped film evaporator.

7. The process as claimed in any one of the preceding claims, **characterized in that** the top stream of the column C1 is recycled to the reaction.

8. The process as claimed in any one of the preceding claims, **characterized in that** the stream separated at the bottom of the film evaporator is recycled at least in part to the reaction.

9. The process as claimed in any one of the preceding claims, **characterized in that** the azeotropic mixture composed of alcohol acrylate with a linear alkyl C₁-C₄ chain and linear C₁-C₄ alcohol generated by the transesterification reaction is recycled to the unit for the synthesis of the alcohol acrylate with a linear alkyl C₁-C₄ chain.

10. The process as claimed in any one of the preceding claims, **characterized in that** the polymerization inhibitor is hydroquinone monomethyl ether (HQME).
